# FASCICULE DE BREVET EUROPEEN

(11) **EP 0 869 750 B1**
(45) Date de publication et mention de la délivrance du brevet: **22.08.2001**
(21) Numéro de dépôt: 96940701.4
(22) Date de dépôt: 19.12.1996
(51) Int. Cl.: A61F 2/06

(54) **ENSEMBLE DE TRAITEMENT CHIRURGICAL D'UNE LUMIERE INTRACORPORELLE**
ANORDNUNG ZUR CHIRURGISCHEN BEHANDLUNG EINES INTRAKORPORALEN KANALS
KIT FOR SURGICAL TREATMENT OF INTRACORPORAL LUMENS

(30) Priorité: 28.12.1995 FR 9515840
(43) Date de publication de la demande: 14.10.1998
(73) Titulaire: SOFRADIM PRODUCTION, 01600 Trévoux (FR)
(72) Inventeur: SGRO, Jean-Claude, F-21000 Dijon (FR)
(74) Mandataire: Guerre, Dominique
(86) Numéro de dépôt international: IB9601465
(87) Numéro de publication internationale: WO9724080

(56) Documents cités:
- EP-A- 0 686 379
- EP-A- 0 689 805
- WO-A-95/07667
- US-A- 5 116 318

## Description

La présente invention concerne le traitement d'une lumière intracorporelle, par application ou mise en place d'une prothèse endoluminale, ou endoprothèse, dans ladite lumière corporelle, par exemple dans une artère ou une veine, dans le domaine de l'angiologie. Et plus particulièrement, l'invention concerne un ensemble de traitement chirurgical facilitant la pose d'une endoprothèse vasculaire dans un vaisseau sanguin.

Selon les méthodes utilisées couramment en chirurgie vasculaire, la mise en place d'endoprothèses vasculaires ou similaires nécessite l'emploi d'un dispositif d'application. Par exemple, les techniques d'introduction et d'application d'endoprothèses, ou expansibles par ballonnet ou auto-expansibles, sont de manière générale bien connues.

Dans le cas d'une endoprothèse expansible, celle-ci est généralement disposée dans sa conformation rétractée, sur et autour d'un ballonnet gonflable, introduite avec le ballonnet par la voie intraveineuse ou intraartérielle, et amenée ainsi sur le site de la lésion ou obstruction à traiter. A cet endroit, le ballonnet est alors gonflé, pour amener l'endoprothèse dans sa conformation déployée, et ainsi traiter la lésion ou obstruction. Les problèmes souvent rencontrés avec un tel système sont liés à la mauvaise retenue de l'endoprothèse sur le ballonnet, ce qui provoque notamment la désolidarisation de l'endoprothèse du ballonnet, ou un largage prématuré de l'endoprothèse, pouvant avoir pour conséquence un mauvais positionnement de cette dernière, ainsi que la possibilité de crevaison du ballonnet par l'une des extrémités de la prothèse, qui est souvent en matière métallique.

Dans le cas d'une endoprothèse auto-expansible, généralement constituée par un matériau à mémoire de forme, celle-ci est souvent introduite dans la veine ou artère, dans deux tubes concentriques qui se chevauchent, donc dans sa conformation rétractée, et le retrait du tube libère l'endoprothèse, et provoque son expansion dans la conformation déployée.

Pour éviter tout déplacement d'une endoprothèse expansible pendant son trajet intraartériel ou intraveineux jusqu'au site d'implantation, il est connu d'utiliser des replis de matière plastique souple, disposés en avant et en arrière du ballonnet, les bords circulaires de la prothèse endoluminale ayant été préalablement glissés en-dessous de ces replis, de sorte qu'elle est retenue par ces derniers et ne peut se déplacer, ni en avant, ni en arrière. Lors du gonflement du ballonnet, la dilatation radiale de ce dernier, et par conséquent de l'endoprothèse, provoque le raccourcissement longitudinal de cette dernière, et permet sa désolidarisation du ballonnet. Par contre, cette solution ne permet pas de contrôler l'application de n'importe quelle prothèse endoluminale, et est en particulier exclue pour les endoprothèses auto-expansibles.

Il était connu également, par le document US-A-5 116 318, de prévoir une enveloppe élastique placée à l'intérieur de l'endoprothèse, pour entourer le ballonnet gonflable servant à effectuer l'angioplastie. L'enveloppe est conçue pour résister à l'expansion radiale du ballonnet, afin d'éviter une rupture de ce dernier, ce qui réduit les risques de traumatisme à la lumière intracorporelle, mais aussi pour se contracter autour du ballonnet, de manière à faciliter le retrait de ce dernier sans accrocher la paroi intérieure de l'endoprothèse.

Une autre solution a été prévue par le document WO-A-95/07667 qui décrit un ballonnet détachable et durcissable associant l'acte d'angioplastie et la pose d'une gaine intérieure, qui agit ensuite comme un stent. Le durcissement chimique se fait par application de chaleur ou de lumière, une fois que le ballonnet a été positionné et gonflé, et son relargage s'effectue par dégradation chimique de zones de séparation constituées de polymères différents de ceux constituant le reste du ballonnet. Toutefois, cette solution ne permet pas de contrôler d'une manière simple le positionnement du ballonnet stent, car les conditions de dégradation chimique et de durcissement qui doivent être appliquées peuvent conduire à un relargage non contrôlé de produits de dégradations potentiellement toxique au niveau local ou systémique.

Il existait alors un besoin de trouver un ensemble de traitement d'une lumière intracorporelle, comportant tous les composants nécessaires à ce traitement sous une forme compacte, et "emballés" en quelque sorte de manière à pouvoir être déployés, avec précision et sans risques pour le malade, de manière facile et contrôlable.

La présente invention résout ce problème en prévoyant un ensemble de traitement d'une lumière intracorporelle, permettant une introduction et mise en place d'une endoprothèse, de manière à la fois sure et simple.

Un ensemble de traitement selon l'invention comprend donc :
- la prothèse endoluminale, ou endoprothèse, généralement de forme tubulaire, susceptible de prendre deux conformations, à savoir l'une rétractée de section interne relativement faible, et l'autre déployée de section interne relativement importante ; cette prothèse peut être, ou expansible sous l'action d'un moyen rapporté et spécifique, ou auto-expansible ;
- des moyens filiformes ou allongés d'introduction de la prothèse à l'intérieur de la lumière corporelle, par exemple du type cathéter ;
- des moyens de dilatation radiale, par exemple un ballonnet gonflable, disposés à une extrémité distale des moyens d'introduction précités, entre ces derniers et la prothèse dans sa conformation rétractée, ces moyens étant agencés pour contrôler, c'est-à-dire provoquer ou accompagner, le passage de la prothèse de sa conformation rétractée à sa conformation déployée ; ces moyens sont actionnés à une extrémité proximale des moyens d'introduction précités, pour être activés ou désactivés sous la commande de l'utilisateur ou manipulateur ;
- et une enveloppe entourant la prothèse dans sa conformation rétractée.

Conformément à l'invention, l'enveloppe est rassemblée et solidaire, directement ou indirectement, à au moins une extrémité, des moyens d'introduction, par exemple du type cathéter. Cette enveloppe comporte une gaine souple s'étendant longitudinalement selon l'axe de la prothèse, entourant cette dernière selon au moins une partie de sa longueur, et susceptible de prendre deux conformations, l'une ramassée correspondant à la conformation rétractée de la prothèse, et l'autre détendue, venant en contact externe avec la paroi intérieure de la lumière intracorporelle traitée, et correspondant à et limitant la conformation déployée de la prothèse ; le passage de la conformation ramassée à la conformation détendue de la gaine s'effectue sous l'effet du passage de la prothèse, de sa conformation rétractée à sa conformation déployée. Et l'enveloppe intègre ou comporte des moyens de séparation mécaniques de la gaine, dans sa conformation détendue, par rapport à au moins une extrémité de l'enveloppe.

Dans la conformation déployée de la prothèse, correspondant à la conformation détendue de la gaine, cette dernière se trouve disposée entre, du côté intérieur la prothèse proprement dite, et du côté extérieur la paroi de la lumière corporelle traitée. De cette manière, les orifices, ouvertures ou mailles de l'endoprothèse se trouvent obturés par la gaine dans sa conformation détendue, ce qui évite toute prolifération de la couche cellulaire à l'intérieur de la lumière intracorporelle, appelée hyperplasie intimale dans le cas d'une artère, et par conséquent toute oblitération de l'endoprothèse, car les cellules proliférantes ne peuvent passer au travers des mailles ou ouvertures de l'endoprothèse. Il s'agit là d'un avantage essentiel et inattendu, en faveur d'un ensemble de traitement chirurgical selon l'invention.

Par ailleurs, la gaine dans sa conformation détendue protège en quelque sorte la paroi de la lumière intracorporelle, vis-à-vis de l'endoprothèse.

La présente invention sera maintenant décrite de manière plus détaillée en se référant au dessin annexé, dans lequel :
- la **Figure 1** représente une vue en perspective avec arrachement partiel d'un ensemble de traitement d'une lumière intracorporelle, conforme à l'invention, avant son introduction dans ladite lumière, et avant l'actionnement des moyens de dilatation radiale ;
- la **Figure 2** représente une vue en perspective avec arrachement partiel de l'ensemble selon Figure 1, après introduction dans la lumière corporelle, à l'endroit du traitement souhaité, et après actionnement des moyens de dilatation radiale ;
- la **Figure 3** représente une vue en coupe axiale de l'ensemble selon Figure 2, montrant ses différents éléments ;
- la **Figure 4** représente une vue en coupe de la prothèse à l'état déployé conformément à la Figure 3, après désactivation des moyens de dilatation radiale, et retrait de ceux-ci avec les moyens d'introduction ;
- la **Figure 5** représente une vue en coupe transversale d'une lumière intracorporelle traitée, dans laquelle est introduit l'ensemble conforme à la Figure 1, avant l'actionnement des moyens de dilatation radiale ;
- la **Figure 6** est une vue similaire à la Figure 5, montrant l'ensemble conforme à la figure 2, après actionnement des moyens de dilatation radiale ;
- la **Figure 7** est une autre vue similaire à celle de la Figure 6, une fois que les moyens d'introduction et de dilatation radiale ont été retirés ;
- la **Figure 8** est une vue en perspective d'une première variante d'un ensemble de traitement selon l'invention, avant son introduction dans la lumière intracorporelle, et actionnement des moyens de dilatation radiale ;
- la **Figure 9** est une vue en perspective de l'ensemble selon Figure 8, après actionnement des moyens de dilatation radiale, sous entendu dans la lumière intracorporelle traitée, mais non représentée ;
- la **Figure 10** est une vue en perspective d'une deuxième variante d'un ensemble selon l'invention, avant actionnement des moyens de dilatation radiale ;
- la **Figure 11** est une vue en perspective de l'ensemble selon Figure 10, après actionnement des moyens de dilatation radiale ;
- la **Figure 12** est une vue en perspective d'une troisième variante d'un ensemble de traitement selon l'invention, avant actionnement des moyens de dilatation radiale ;
- la **Figure 13** est une vue en coupe axiale de l'ensemble de traitement représenté à la Figure 12, avant actionnement des moyens de dilatation radiale ;
- la **Figure 14** est une vue en coupe axiale de l'ensemble de traitement représenté à la Figure 12, après introduction dans une lumière corporelle et actionnement des moyens de dilatation radiale ;
- la **Figure 15** est une vue en perspective d'une modification préférée d'un ensemble de traitement conforme aux Figures 12 à 14, avant actionnement des moyens de dilatation radiale ;
- la **Figure 16** est une vue en coupe de l'ensemble de traitement selon Figure 15, après actionnement des moyens de dilatation radiale ;
- la **Figure 17** est une vue en coupe de l'ensemble de traitement selon Figures 15 et 16, en place, dans une lumière intracorporelle, après désactivation des moyens de dilatation radiale, et retrait de ceux-ci avec les moyens d'introduction.

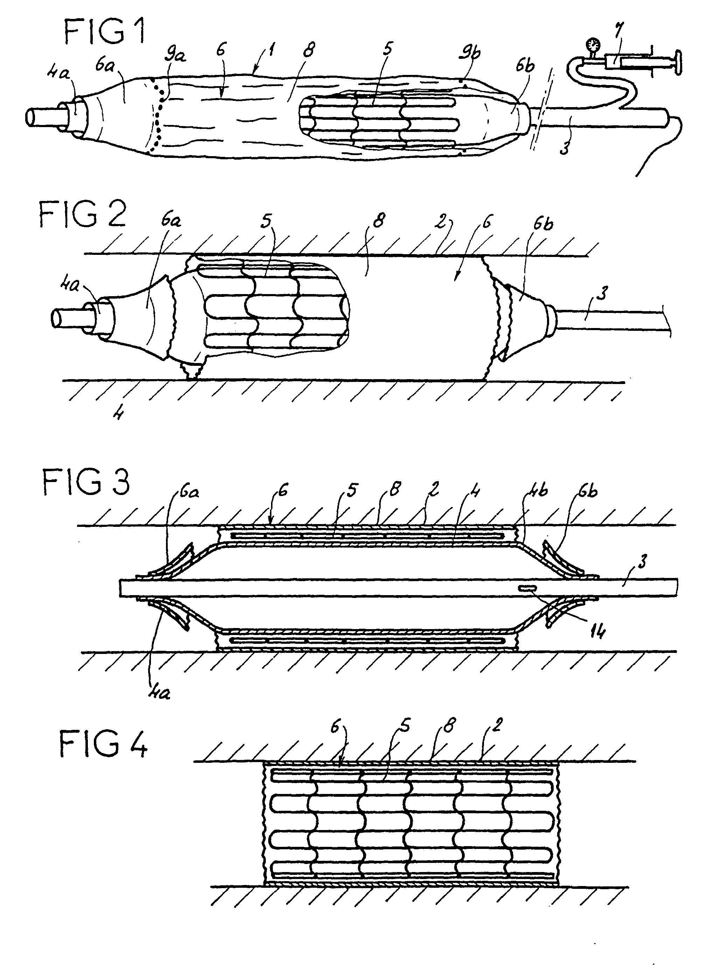

Par référence aux Figures 1 à 7, on décrit maintenant un ensemble 1 de traitement chirurgical d'une lumière 2 intracorporelle, conforme à l'invention.

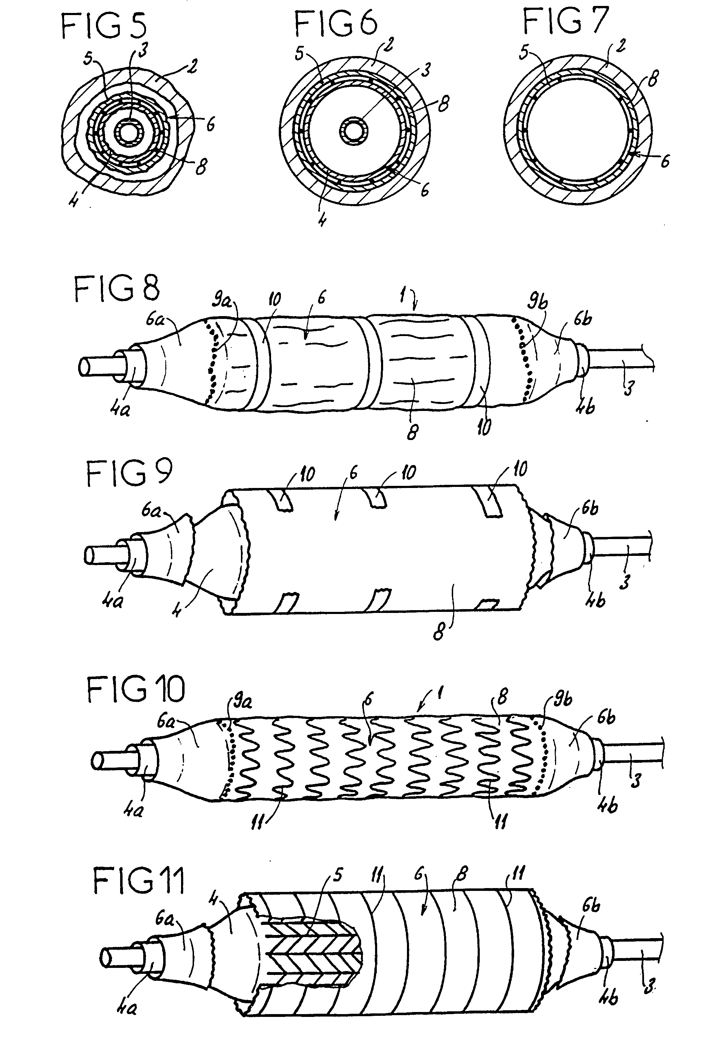

Un ensemble 1 selon l'invention comprend :
- une prothèse 5 endoluminale, de type traditionnel, expansible ou auto-expansible, généralement de forme tubulaire, et réalisée ou obtenue par exemple à partir d'un fil métallique biocompatible ; cette prothèse 5 est susceptible de prendre deux conformations, à savoir l'une rétractée, montrée aux Figures 1 et 5, de section interne relativement faible, et l'autre déployée, montrée aux Figures 2 à 4, et 6 et 7, de section interne relativement importante ;
- des moyens 3 filiformes ou allongés d'introduction de la prothèse 5 à l'intérieur de la lumière corporelle 2 ; conformément à la Figure 1, il s'agit par exemple d'un cathéter traditionnel, permettant également une endoscopie ;
- des moyens 4 de dilatation radiale, disposés à une extrémité distale des moyens 3 d'introduction, entre ces derniers et la prothèse 5 dans sa conformation rétractée, et agencés pour contrôler comme décrit ci-après le passage de la prothèse 5 de sa conformation rétractée à sa conformation déployée ; il s'agit, comme représenté à la figure 3, d'un ballonnet gonflable, dont les deux extrémités 4a et 4b sont solidarisées de manière étanche sur les moyens d'introduction 3, en l'occurrence le cathéter ;
- des moyens d'actionnement 7 des moyens 4 de dilatation radiale, disposés à une extrémité proximale des moyens d'introduction 3, comme représenté à la figure 1 ; ces moyens 7 consistent de manière traditionnelle en une pompe, du type seringue, permettant d'injecter un gaz ou un liquide dans le ballonnet gonflable 4, par l'orifice 14 montré à la Figure 3 ; ces mêmes moyens d'actionnement permettent en sens inverse d'extraire du liquide ou du gaz du ballonnet 4 ;
- et une enveloppe 6 entourant la prothèse 5 dans sa conformation rétractée.

Conformément à l'invention, l'enveloppe 6 est tout d'abord rassemblée et solidaire à ses deux extrémités 6a et 6b respectivement, des moyens 3 d'introduction, en l'occurrence du cathéter ; cette solidarisation s'effectue, soit directement au contact du cathéter 3, soit sur les deux extrémités 4a et 4b du ballonnet 4, elles-mêmes solidarisées de manière étanche sur le cathéter 3.

Ensuite, l'enveloppe 6 comporte une gaine 8 souple, d'extension longitudinale selon l'axe de la prothèse 5, entourant cette dernière selon au moins une partie de sa longueur, en l'occurrence la totalité de sa longueur, susceptible de prendre deux conformations, l'une ramassée montrée aux Figures 1 et 5, correspondant à la conformation rétractée de la prothèse 5, et l'autre détendue montrée aux Figures 2 à 4, et 6 et 7, venant au contact externe de la paroi intérieure de la lumière intracorporelle 2, correspondant à et limitant la conformation déployée de la prothèse 5. Le passage de la conformation ramassée à la conformation détendue de la gaine 8 s'effectue sous l'effet du passage de la prothèse 5, de sa conformation rétractée à sa conformation déployée.

Et finalement, l'enveloppe 6 intègre des moyens de séparation mécaniques 9a et 9b de la gaine 8, dans sa conformation détendue, par rapport aux deux extrémités 6a et 6b respectivement de l'enveloppe 6.

Au moins la partie de l'enveloppe 6, formant la gaine 8 se présente sous la forme d'une paroi souple, rassemblée dans sa conformation ramassée, par des plis longitudinaux parallèles à l'axe de la prothèse 5. Au moins la partie de l'enveloppe 6, formant cette gaine 8, est obtenue sous la forme d'une paroi continue et perméable, par exemple sous la forme d'un tissu, d'un film ou d'une nappe, en au moins un matériau biocompatible, et/ou hémocompatible, éventuellement résorbable, notamment en PTFE, polyuréthanne, polyester, polyamide, polypropylène, collagène et les polymères dérivés de l'acide hyaluronique et/ou lactique.

Lorsque la prothèse 5 est expansible sous l'effet des moyens 4 de dilatation radiale, dans la conformation ramassée de la gaine 8, cette dernière demeure libre par rapport à la prothèse 5, elle-même dans sa conformation rétractée.

Lorsque la prothèse 5 est auto-expansible, deux modalités peuvent être adoptées en ce qui concerne la gaine 8 :
- soit dans la conformation ramassée de la gaine 8, cette dernière a une résistance suffisante pour contenir l'auto-expansion radiale en rappel de la prothèse 5, elle-même dans sa conformation rétractée, mais insuffisante pour s'opposer en plus à l'effort radial des moyens 4 de dilatation radiale, activés par les moyens d'actionnement 7 ;
- soit la partie de l'enveloppe 6, formant la gaine 8, est obtenue en un matériau élastique, ou visco-élastique, ayant au départ une épaisseur suffisante pour contenir l'auto-expansion radiale en rappel de la prothèse 5, elle-même dans sa conformation rétractée, puis susceptible de s'amincir sous l'effet de l'effort radial exercé par les moyens 4 de dilatation radiale, en libérant ainsi l'auto-expansion radiale de la prothèse 5 vers sa conformation déployée.

Conformément à la représentation de la figure 1, les moyens de séparation mécaniques 9a et 9b consistent ou comprennent deux lignes de faiblesse mécaniques fermées, ayant chacune une forme alternée en zig-zag, s'étendant généralement selon deux circonférences ou bandes respectivement concentriques avec l'axe de la prothèse 5. Ces deux moyens de séparation, ou lignes de faiblesse, par exemple sous la forme de prédécoupes en "pointillés", ménagées dans la paroi ou matériau de l'enveloppe 6, sont disposés de part et d'autre de la gaine 8. Et sous l'effet mécanique de l'expansion radiale de l'enveloppe 6, au contact de la prothèse 5 elle-même en expansion radiale, ces deux lignes de faiblesse 9a et 9b forment deux lignes de séparation complète, comme montré à la Figure 2, lorsque la gaine 8 passe par conséquent de sa conformation ramassée à sa conformation détendue.

La première variante de l'ensemble précédemment décrit, se distingue de ce dernier par le fait que, par référence aux Figures 8 et 9, des bandes circonférentielles 10, éventuellement fermées, sont rapportées et distribuées sur la gaine 8, et agencées pour contenir avec ladite gaine l'auto-expansion radiale en rappel de la prothèse 5, mais aussi pour se rompre sous l'effort radial supplémentaire exercé par les moyens 4 de dilatation radiale, lorsque ces derniers sont activés. Ces mêmes bandes peuvent être intégrées, d'une manière ou d'une autre, dans la paroi de la gaine 8.

Selon la deuxième variante de l'ensemble selon l'invention, représentée aux Figures 10 et 11, la gaine 8 intègre des moyens d'arrêt 11 de sa propre détente, disposés à l'état replié (cf Fig.10) dans la conformation ramassée de ladite gaine, et à l'état déplié (cf Fig.11), dans la conformation détendue de la gaine 8. A l'état déplié, dans la conformation détendue de la gaine 8, les moyens d'arrêt 11 sont agencés ou choisis pour résister à toute poussée radiale, exercée par exemple par une endoprothèse auto-expansible, au-delà ou supérieure à celle nécessaire ou consécutive au passage de la prothèse 5, de sa conformation rétractée à sa conformation déployée. Comme représenté aux Figures 10 et 11, ces moyens d'arrêt 11 sont constitués par des fils, ou tous autres moyens filiformes, notamment des fibres, agencés circonférentiellement de manière continue, et constitués par au moins un matériau résistant mécaniquement, notamment polyester, polyuréthanne, polypropylène. A l'état replié, chacun de ces fils a une forme alternée en zig-zag, et à l'état déplié chacun de ces fils prend une forme circulaire.

Cette deuxième variante peut être utilisée pour traiter des régions anevrysmales, c'est-à-dire dilatées à paroi très fragile, puisqu'elle permet une limitation de la détente de la gaine 8. En particulier, la dilatation du ballonnet 4 se heurte à la résistance exercée par les moyens d'arrêt 11, à l'état déplié, ce qui évite aussi une poursuite de la détente de la gaine 8 sous l'effet de la pression du sang, dans le cas d'un traitement chirurgical vasculaire.

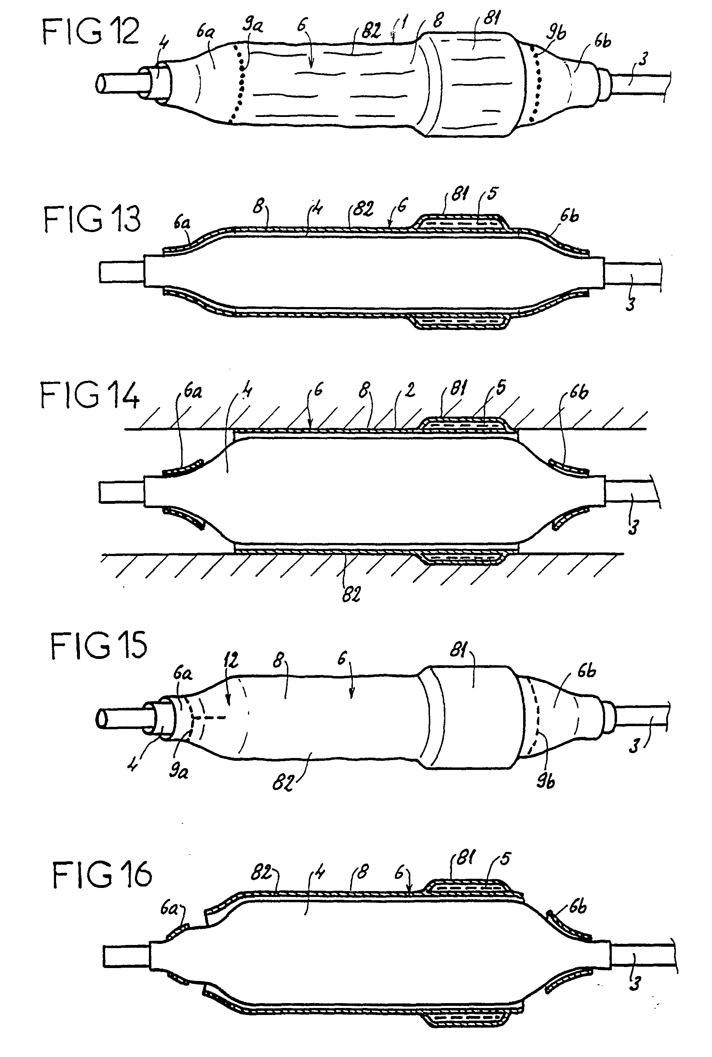

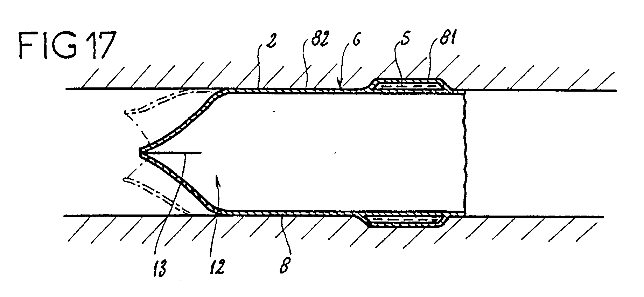

La troisième variante de l'ensemble selon l'invention, représentée par référence aux Figures 12 à 17, se caractérise par le fait que la gaine 8 comprend deux parties, à savoir une première partie 81 entourant la prothèse 5, et une deuxième partie 82, non résorbable, agencée à la fois pour former une valvule 12, dans la conformation détendue de la gaine 8, et demeurée libre sous cette forme à l'intérieur de la lumière intracorporelle 2 traitée. Dans la conformation détendue de la gaine 8, la deuxième partie 82 a éventuellement une section plus faible que celle de la section de la première partie 81, dans la conformation détendue de la gaine 8 ; cette section plus faible est en fait décroissante, sous forme conique, de la première partie 81 vers l'extrémité opposée à celle-ci.

La deuxième partie 82 de la gaine 8 présente, dans la conformation détendue de cette dernière, deux fentes 13 opposées, parallèles à l'axe de la prothèse 5, obtenues à partir de deux lignes correspondantes de faiblesse, représentées à la figure 15, et ménagées dans la deuxième partie 82, dans la conformation ramassée de la gaine 8. Les deux lignes partielles 13 de faiblesse sont disposées perpendiculairement à la ligne de faiblesse 9a, permettant de séparer la gaine 8 de l'extrémité 6a de l'enveloppe 6.

Par ailleurs, la première partie 81 forme en fait un repli permettant d'enserrer complètement la prothèse 5, comme représenté aux Figures 13, 14, 16 et 17.

Cette troisième variante permet d'adjoindre un système valvulaire à l'endoprothèse, pouvant être utilisé dans le traitement chirurgical de vaisseaux sanguins veineux, par exemple. Ceci permet en particulier de traiter les veines se reperméabilisant, dont les valvules ne fonctionnent plus, après des épisodes de phlébite profonde et importante, sans avoir à prélever des veines normales du même individu ou patient, et les implanter chirurgicalement en lieu et place des veines défaillantes. Ceci permet aussi de traiter certains cas de varices dues à une incontinence valvulaire, en gardant le potentiel veineux.

Selon cette variante, l'endoprothèse est essentiellement utilisée pour bloquer le système valvulaire prothétique en place dans le vaisseau sanguin.

## Revendications

1. Ensemble (1) de traitement d'une lumière (2) intracorporelle comprenant une prothèse (5) endoluminale, généralement de forme tubulaire, susceptible de prendre deux conformations, à savoir l'une rétractée (Fig. 1,8,10,12) de section interne relativement faible, et l'autre déployée (Fig. 2,9,11,14), de section interne relativement importante, des moyens (3) filiformes d'introduction à l'intérieur de la lumière corporelle (2), notamment un cathéter, des moyens (4) de dilatation radiale, notamment un ballonnet gonflable, disposés à une extrémité distale des moyens (3) d'introduction, entre ces derniers et la prothèse (5) dans sa conformation rétractée, agencés pour contrôler le passage de ladite prothèse de sa conformation rétractée à sa conformation déployée, et actionnés (7) à une extrémité proximale des moyens d'introduction (3), et une enveloppe (6) entourant la prothèse (5) dans sa conformation rétractée, **caractérisé en ce que** l'enveloppe (6) est rassemblée et solidaire à au moins une extrémité (6a,6b) des moyens (3) d'introduction, et comporte une gaine (8) souple s'étendant longitudinalement selon l'axe de la prothèse (5), entourant cette dernière selon au moins une partie de sa longueur, susceptible de prendre deux conformations, l'une ramassée (Fig. 1,8,10,12) correspondant à la conformation rétractée de la prothèse (5), et l'autre détendue (Fig. 2,9,11,14) venant au contact externe de la paroi intérieure de la lumière intracorporelle (2), correspondant à et limitant la conformation déployée de ladite prothèse, le passage de la conformation ramassée à la conformation détendue s'effectuant sous l'effet du passage de ladite prothèse (5) de sa conformation rétractée à sa conformation déployée, et l'enveloppe (6) intègre des moyens de séparation mécaniques (9a,9b) de la gaine (8), dans sa conformation détendue, par rapport à au moins une extrémité (6a,6b) de ladite enveloppe.

2. Ensemble selon la revendication 1, caractérisé en ce que les moyens de séparation mécaniques (9a,9b) comprennent deux lignes de faiblesse mécaniques fermées, s'étendant selon deux circonférences ou bandes concentriques avec l'axe de la prothèse (5), de part et d'autre de la gaine (8), susceptibles de former respectivement deux lignes de séparation complète (Fig. 2,9,11,14) lorsque la gaine (8) passe de sa conformation ramassée à sa conformation détendue.

3. Ensemble selon la revendication 1, caractérisé en ce qu'au moins la partie de l'enveloppe (6), formant la gaine (8) est obtenue, par exemple sous la forme d'un tissu, d'un film ou d'une nappe, en au moins un matériau biocompatible, et/ou hémocompatible, éventuellement résorbable, notamment en PTFE, polyuréthanne, polyester, polyamide, polypropylène, collagène, et les polymères dérivés de l'acide hyaluronique et/ou lactique.

4. Ensemble selon la revendication 1, selon lequel la prothèse (5) est expansible sous l'effet des moyens (4) de dilatation radiale, caractérisé en ce que, dans la conformation ramassée de la gaine (8), cette dernière est libre par rapport à la prothèse, dans sa conformation rétractée.

5. Ensemble selon la revendication 1, selon lequel la prothèse (5) est auto-expansible, caractérisé en ce que, dans la conformation ramassée de la gaine, cette dernière a une résistance suffisante pour contenir l'auto-expansion radiale en rappel de la prothèse, elle-même dans sa conformation rétractée, mais insuffisante pour s'opposer en plus à l'effort radial des moyens (4) de dilatation radiale.

6. Ensemble selon la revendication 5, caractérisé en ce que des bandes circonférentielles (10), éventuellement fermées, sont rapportées et distribuées sur la gaine (8), et agencées pour contenir avec ladite gaine l'auto-expansion radiale en rappel de la prothèse, mais pour se rompre sous l'effort radial supplémentaire exercé par les moyens (4) de dilatation radiale.

7. Ensemble selon la revendication 5, caractérisé en ce qu'au moins la partie de l'enveloppe (6), formant la gaine (8), est obtenue en un matériau élastique, ayant au départ une épaisseur suffisante pour contenir l'auto-expansion radiale en rappel de la prothèse (5), elle-même dans sa conformation rétractée, puis susceptible de s'amincir sous l'effet de l'effort radial exercé par les moyens (4) de dilatation radiale, en libérant ladite prothèse vers sa conformation déployée.

8. Ensemble selon la revendication 1, caractérisé en ce que la gaine (8) intègre des moyens d'arrêt (11) de la détente de la gaine, à l'état replié (Fig.10) dans la conformation ramassée de ladite gaine, et à l'état déplié (Fig.11) dans la conformation détendue de ladite gaine, mais résistant dans ce dernier état à toute poussée radiale au-delà de celle nécessaire au ou consécutive au passage de la prothèse de sa conformation rétractée à sa conformation déployée.

9. Ensemble selon la revendication 8, caractérisé en ce que les moyens d'arrêt (11) comprennent des fils, ou des fibres, ou autres moyens filiformes, agencés circonférentiellement de manière continue, et en au moins un matériau résistant, notamment polyester, polyuréthanne, polypropylène.

10. Ensemble selon la revendication 1, caractérisé en ce que la gaine (8) comprend deux parties, à savoir une première partie (81) entourant la prothèse (5), et une deuxième partie (82) agencée pour demeurer libre à l'intérieur de la lumière intracorporelle (2) et ayant éventuellement dans la conformation détendue de la gaine (8) une section plus petite que celle de la première partie, toujours dans la conformation détendue de ladite gaine.

11. Ensemble selon la revendication 10, caractérisé en ce que la deuxième partie (82) de la gaine (8), non résorbable, est agencée pour former une valvule (12), dans la conformation détendue de la gaine.

12. Ensemble selon la revendication 10, caractérisé en ce que la deuxième partie (82) de la gaine (8) présente, dans la conformation détendue de cette dernière, une section décroissante de la première partie (81) vers l'extrémité opposée.

13. Ensemble selon la revendication 12, caractérisé en ce que la partie (82) de la gaine (8) présente, dans la conformation détendue de cette dernière, deux fentes (13). opposées parallèles à l'axe de la prothèse (5), obtenues à partir de deux lignes correspondantes de faiblesse, ménagées dans ladite deuxième partie, dans la conformation ramassée de la gaine (8).

## Patentansprüche

1. Anordnung (1) zur Behandlung eines intrakorporalen Lumens (2), mit einer allgemein rohrförmigen endoluminalen Prothese (5), die in der Lage ist, zwei Zustände einzunehmen, nämlich einen zusammengezogenen (Fig. 1, 8, 10, 12) von relativ geringem Innenquerschnitt, und einen ausgebreiteten (Fig. 2, 9, 11, 14) von relativ großem Innenquerschnitt, fadenförmigen Einführmitteln (3) zum Einführen in das Innere des korporalen Lumens (2), insbesondere einem Katheter, Dilatationsmitteln (4) zum radialen Dilatieren, insbesondere einem aufblähbaren kleinen Ballon, die an einem distalen Ende der Einführmittel (3) zwischen letzteren und der Prothese (5) in ihrem zusammengezogenen Zustand angeordnet sind, und die dazu ausgestaltet sind, den Übergang der Prothese von ihrem zusammengezogenen Zustand in ihren ausgebreiteten Zustand zu kontrollieren, und die an einem proximalen Ende der Einführmittel (3) betätigt werden (7), und mit einer Umhüllung (6), die die Prothese (5) in ihrem zusammengezogenen Zustand umgibt, dadurch gekennzeichnet, daß die Umhüllung (6) zusammengefügt und mit zumindest einem Ende (6a, 6b) mit den Einführmitteln verbunden ist und eine sich gemäß der Achse der Prothese (5) längs erstreckende und letztere über zumindest einen Teil ihrer Länge umgebende biegsame Schutzhülle (8) aufweist, die in der Lage ist, zwei Zustände einzunehmen, einen gerafften (Fig. 1, 8, 10, 12), der dem zusammengezogenen Zustand der Prothese (5) entspricht, und einen ausgedehnten (Fig. 2, 9, 11, 14), in dem sie mit der Innenwand des intrakorporalen Lumens (2) in Kontakt kommt, und der dem ausgebreiteten Zustand der Prothese entspricht und diesen begrenzt, wobei der Übergang von dem gerafften Zustand in den ausgedehnten Zustand unter der Wirkung des Übergangs der Prothese (5) von ihrem zusammengezogenen Zustand in ihren ausgebreiteten Zustand erfolgt, und daß in der Umhüllung (6) mechanische Trennmittel (9a, 9b) zum Abtrennen der Schutzhülle (8) in ihrem ausgedehnten Zustand bezüglich zumindest einem Ende (6a, 6b) der Umhüllung integriert sind.

2. Anordnung nach Anspruch 1, dadurch gekennzeichnet, daß die mechanischen Trennmittel (9a, 9b) zwei mechanische geschlossene Schwächungslinien aufweisen, die sich beidseits der Schutzhülle (8) gemäß zwei zu der Achse der Prothese (5) konzentrischen Umfängen bzw. Bändern erstrecken, und die in der Lage sind, jeweils zwei Linien für eine vollständige Trennung (Fig. 2, 9, 11, 14) zu bilden, wenn die Schutzhülle (8) von ihrem gerafften Zustand in ihren ausgedehnten Zustand übergeht.

3. Anordnung nach Anspruch 1, dadurch gekennzeichnet, daß zumindest der Teil der Umhüllung (6), der die Schutzhülle (8) bildet, beispielsweise in Form eines Gewebes, eines Films oder einer Lage aus zumindest einem biokompatiblen und/oder hämokompatiblen, ggf. resorbierbaren Material, insbesondere aus PTFE, Polyurethan, Polyester, Polyamid, Polypropylen, Kollagen, und aus Polymeren, die Derivate von Hyaluron- und/oder Milchsäure sind, gefertigt ist.

4. Anordnung nach Anspruch 1, gemäß der die Prothese (5) unter der Wirkung der Dilatationsmittel (4) zum radialen Dilatieren expandierbar ist, dadurch gekennzeichnet, daß im gerafften Zustand der Schutzhülle (8) letztere bezüglich der Prothese in ihrem zusammengezogenen Zustand frei ist.

5. Anordnung nach Anspruch 1, gemäß der die Prothese (5) selbst-expandierbar ist, dadurch gekennzeichnet, daß im gerafften Zustand der Schutzhülle letztere einen ausreichenden mechanischen Widerstand aufweist, um die radiale Selbstexpansion der Prothese zurückzuhalten, wenn diese im zusammengezogenen Zustand ist, der jedoch nicht ausreichend ist, um sich darüber hinaus der radialen Kraft der Dilatationsmittel (4) zum radialen Dilatieren entgegenzusetzen.

6. Anordnung nach Anspruch 5, dadurch gekennzeichnet, daß, ggf. geschlossene, umfängliche Bänder (10) an die Schutzhülle (8) angefügt und auf dieser verteilt sind, die dazu ausgestaltet sind, mit der Schutzhülle die radiale Selbstexpansion der Prothese zurückzuhalten, jedoch unter der durch die Dilatationsmittel (4) zum radialen Dilatieren ausgeübten zusätzlichen radialen Kraft aufzubrechen.

7. Anordnung nach Anspruch 5, dadurch gekennzeichnet, daß zumindest der Teil der Umhüllung (6), der die Schutzhülle (8) bildet, aus einem elastischen Material gefertigt ist, das anfänglich eine Dicke aufweist, die ausreichend ist, um die radiale Selbstexpansion der Prothese (5), wenn diese im zusammengezogenen Zustand ist, zurückzuhalten, und die dann in der Lage ist, unter der Wirkung der durch die Dilatationsmittel (4) zum radialen Dilatieren ausgeübten radialen Kraft dünner zu werden, wodurch die Prothese in ihren ausgebreiteten Zustand freigegeben wird.

8. Anordnung nach Anspruch 1, dadurch gekennzeichnet, daß in der Schutzhülle (8) Stopmittel (11) zum Stoppen der Ausdehnung der Schutzhülle (8), die im gerafften Zustand der Schutzhülle in einem zusammengefalteten Zustand (Fig. 10) und im ausgedehnten Zustand der Schutzhülle (8) in einem entfalteten Zustand sind (Fig. 11), integriert sind, die sich jedoch im letzteren Zustand jeglichem radialen Druck, der über den für den Übergang der Prothese von ihrem zusammengezogenen Zustand in ihren ausgebreiteten Zustand erforderlichen Druck hinausgeht, oder der die Folge dessen ist, entgegensetzen.

9. Anordnung nach Anspruch 8, dadurch gekennzeichnet, daß die Stopmittel (11) Fäden oder Fasern oder andere fadenförmige Mittel aufweisen, die in kontinuierlicher Weise umfänglich angeordnet sind und aus zumindest einem widerstandsfähigen Material, insbesondere Polyester, Polyurethan, Polypropylen, gebildet sind.

10. Anordnung nach Anspruch 1, dadurch gekennzeichnet, daß die Schutzhülle (8) zwei Teile aufweist, nämlich einen ersten Teil (81), der die Prothese (5) umgibt, und einen zweiten Teil (82), der so ausgestaltet ist, daß er im Inneren des intrakorporalen Lumens (2) frei bleibt, und der ggf. im ausgedehnten Zustand der Schutzhülle (8) einen kleineren Querschnitt als der erste Teil aufweist, jedenfalls im ausgedehnten Zustand der Schutzhülle.

11. Anordnung nach Anspruch 10, dadurch gekennzeichnet, daß der nicht resorbierbare zweite Teil (82) der Schutzhülle (8) so ausgestaltet ist, daß er eine Klappe (12) im ausgedehnten Zustand der Schutzhülle (8) bildet.

12. Anordnung nach Anspruch 10, dadurch gekennzeichnet, daß der zweite Teil (82) der Schutzhülle (8) im ausgedehnten Zustand letzterer einen von dem ersten Teil (81) zu dem gegenüberliegenden Ende abnehmenden Querschnitt aufweist.

13. Anordnung nach Anspruch 12, dadurch gekennzeichnet, daß der Teil (82) der Schutzhülle (8) im ausgedehnten Zustand letzterer zwei zur Achse der Prothese (5) parallele gegenüberliegende Schlitze (13) aufweist, die aus zwei entsprechenden Schwächungslinien gebildet sind, die in dem zweiten Teil im gerafften Zustand der Schutzhülle (8) ausgebildet sind.

## Claims

1. Assembly (1) for the treatment of an intracorporeal lumen (2), comprising an intraluminal prosthesis (5), generally of tubular shape and capable of adopting two configurations, namely a retracted one (Fig. 1, 8, 10, 12) of relatively small internal cross section, and a deployed one (Fig. 2, 9, 11, 14) of relatively large internal cross section, filamentous means (3) for introduction into the corporeal lumen (2), in particular a catheter, radial expansion means (4), in particular an inflatable balloon, arranged at a distal end of the introduction means (3), between the latter and the prosthesis (5) in its retracted configuration, are designed to control the transition of the said prosthesis from its retracted configuration to its deployed configuration, and are actuated (7) at a proximal end of the introduction means (3), and an envelope (6) which surrounds the prosthesis (5) in its retracted configuration, characterized in that the envelope (6) is gathered and secured at least at one end (6a, 6b) to the introduction means (3), and has a flexible sheath (8) which extends longitudinally along the axis of the prosthesis (5), surrounds the latter over at least a part of its length, and can adopt two configurations, a contracted one (Fig. 1, 8, 10, 12) corresponding to the retracted configuration of the prosthesis (5), and an extended one (Fig. 2, 9, 11, 14) coming into external contact with the internal wall of the intracorporeal lumen (2), and corresponding to and limiting the deployed configuration of the said prosthesis, the transition from the contracted configuration to the extended configuration taking place under the effect of the transition of the said prosthesis (5) from its retracted configuration to its deployed configuration, and the envelope (6) incorporates mechanical means (9a, 9b) for separating the sheath (8), in its extended configuration, from at least one end (6a, 6b) of the said envelope.

2. Assembly according to Claim 1, characterized in that the mechanical separation means (9a, 9b) comprise two closed mechanically weak lines which extend along two circumferences or bands concentric with the axis of the prosthesis (5), on either side of the sheath (8), and can respectively form two lines of full separation (Fig. 2, 9, 11, 14) when the sheath (8) changes from its contracted configuration to its extended configuration.

3. Assembly according to Claim 1, characterized in that at least the part of the envelope (6) forming the sheath (8) is obtained, for example in the form of a fabric, a film or a cloth, from at least one optionally absorbable biocompatible and/or haemocompatible material, in particular from PTFE, polyurethane, polyester, polyamide, polypropylene, collagen and polymers derived from hyaluronic and/or lactic acid.

4. Assembly according to Claim 1, according to which the prosthesis (5) can expand under the effect of the radial expansion means (4), characterized in that, when the sheath is in the contracted configuration, it is free relative to the prosthesis, which is in its retracted configuration.

5. Assembly according to Claim 1, according to which the prosthesis (5) is autoexpansible, characterized in that, when the sheath is in the contracted configuration, it is strong enough to contain the return radial autoexpansion of the prosthesis, itself in its retracted configuration, but not strong enough to additionally counter the radial force of the radial expansion means.

6. Assembly according to Claim 5, characterized in that optionally closed circumferential bands (10) are attached and distributed over the sheath (8), and are designed to contain with the said sheath the return radial autoexpansion of the prosthesis, but to break under the additional radial force exerted by the radial expansion means (4).

7. Assembly according to Claim 5, characterized in that at least the part of the envelope (6) forming the sheath (8) is obtained from an elastic material, which initially has a thickness sufficient to contain the return radial autoexpansion of the prosthesis (5), itself in its retracted configuration, then can become thinner under the effect of the radial force exerted by the radial expansion means (4), releasing the said prosthesis into its deployed configuration.

8. Assembly according to Claim 1, characterized in that the sheath (8) incorporates means (11) for stopping the extension of the sheath, in the folded state (Fig. 10) when the said sheath is in the contracted configuration, and in the unfolded state (Fig. 11) when the said sheath is in the extended configuration, but in the latter state withstanding any radial thrust beyond that necessary for or due to the transition of the prosthesis from its retracted configuration to its deployed configuration.

9. Assembly according to Claim 8, characterized in that the stop means (11) comprise threads, or fibres, or other filamentous means, arranged circumferentially in continuous fashion and made of at least one strong material, in particular polyester, polyurethane or polypropylene.

10. Assembly according to Claim 1, characterized in that the sheath (8) comprises two parts, namely a first part (81) which surrounds the prosthesis (5), and a second part (82) which is designed to remain free inside the intracorporeal lumen (2) and optionally has, when the sheath (8) is in the extended configuration, a smaller cross section than that of the first part, still when the said sheath is in the extended configuration.

11. Assembly according to Claim 10, characterized in that the second part (82) of the sheath (8), which is not absorbable, is designed to form a valve (12) when the sheath is in the extended configuration.

12. Assembly according to Claim 10, characterized in that the second part (82) of the sheath (8) has, when the latter is in the extended configuration, a cross section which decreases from the first part (81) to the opposite end.

13. Assembly according to Claim 12, characterized in that the part (82) of the sheath (8) has, when the latter is in the extended configuration, two opposite slits (13) which are parallel to the axis of the prosthesis (5) and are obtained from two corresponding weak lines that are arranged in the said second part when the sheath (8) is in the contracted configuration.
